# EUROPÄISCHE PATENTSCHRIFT

(11) **EP 1 272 449 B1**
(45) Veröffentlichungstag und Bekanntmachung des Hinweises auf die Patenterteilung: **21.09.2005**
(21) Anmeldenummer: 01931538.1
(22) Anmeldetag: 19.03.2001
(51) Int. Cl.: C07C 39/16, C07C 37/68

(54) **STOFFGEMISCH ENTHALTEND BISPHENOL A**
SUBSTANCE MIXTURE CONTAINING BISPHENOL A
MELANGE DE SUBSTANCES CONTENANT DU BISPHENOL A

(30) Priorität: 30.03.2000 DE 10015864
(43) Veröffentlichungstag der Anmeldung: 08.01.2003
(73) Patentinhaber: Bayer MaterialScience AG, 51368 Leverkusen (DE)
(72) Erfinder: BÖDIGER, Michael, League City, TX 77573 (US); NEUMANN, Rainer, 47803 Krefeld (DE); HEYDENREICH, Frieder, 40593 Düsseldorf (DE); PREIN, Michael, B-2930 Brasschaat (BE)
(86) Internationale Anmeldenummer: PCT/EP2001/003114
(87) Internationale Veröffentlichungsnummer: WO 2001/074750

(56) Entgegenhaltungen:
- EP-A- 0 552 518
- EP-A- 0 758 637
- EP-A- 0 812 815

## Beschreibung

Die vorliegende Erfindung betrifft Stoffgemische enthaltend Bisphenol A sowie zwei Verfahren zu deren Herstellung sowie deren Verwendung zur Herstellung von Polymerwerkstoffen.

Bis(4-hydroxyaryl)alkane, im Folgenden Bisphenole genannt, sind als Ausgangsstoffe oder als Zwischenprodukte zur Herstellung einer Vielzahl kommerzieller Produkt von Bedeutung. Bisphenole können durch die Kondensation von Phenolen und Carbonylverbindungen hergestellt werden. Dabei können substituierte Phenole oder unsubstituiertes Phenol verwendet werden.

Von besonderer technischer Bedeutung ist das Kondensationsprodukt aus der Reaktion zwischen Phenol und Aceton, 2,2-Bis(4-hydroxyphenyl)propan (Bisphenol A, BPA, p,p-BPA). BPA dient als Ausgangstoff zur Herstellung verschiedenartiger Polymerwerkstoffe wie beispielsweise Polyarylate, Polyetherimide, Polysulfone und modifizierter Phenol-Formaldehydharze. Bevorzugte Anwendungsgebiete liegen in der Herstellung von Epoxyharzen und Polycarbonaten.

Verfahren zur Herstellung von Bisphenolen durch sauerkatalysierte Umsetzung von Phenolen mit Carbonylverbindungen sind beispielsweise bekannt aus EP-A-0 552 518, US-A 2 775 620 und aus EP-A-0 342 758.

Bisphenole allgemeiner Struktur können nach Verfahren hergestellt werden, die den Verfahren zur Herstellung von BPA analog sind.

Phenolharze sind Kunstharze, die durch Kondensation von Phenolen (bzw. Bisphenol A) mit Aldehyden, insbesondere Formaldehyd, durch Derivatisierung der dabei resultierenden Kondensate oder durch Addition von Phenolen an ungesättigten Verbindungen, wie z.B. Acetylen, Terpene oder natürliche Harze gewonnen werden.

Als Epoxidharze, bezeichnet man sowohl oligomere Verbindungen mit mehr als einer Epoxidgruppe pro Mol, die zur Herstellung von Duroplasten eingesetzt werden, als auch die entsprechenden Duroplasten selbst. Die Umwandlung der Epoxidharze erfolgt über Polyadditionsreaktionen mit geeigneten Härtern bzw. durch Polymerisation über die Epoxid-Gruppe. Über 90 % der heutigen Weltproduktion erfolgt durch Umsetzung von Bisphenol A mit Epichlorhydrin.

Unter dem Begriff Formaldehydharze werden die techn. sehr wichtigen Harnstoff-, Melamin-, Phenol- und im weiteren Sinne auch die Furanharze zusammengefasst, die durch Kondensation von Formaldehyd mit Harnstoff, Melamin, Phenol oder Phenolen (u.a. Bisphenol A) und Fufurylalkohol als NH- bzw. OH-Gruppen enthaltenen Monomere hergestellt werden.

Poymerwerkstoffe wie beispielsweise Phenolharze, Epoxidharze oder Formaldehydharze können unter Verwendung von Bisphenol A als einem Rohstoff hergestellt werden. Nachteilig dabei ist, dass Bisphenol A in reiner Form teuer ist, ausserdem ist nachteilig, dass das Eigenschaftsniveau der genannten Polymerwerkstoffe, wenn sie unter Verwendung von reinem Bisphenol A als einem Rohstoff hergestellt werden, nicht optimal ist.

Der vorliegenden Erfindung liegt somit die Aufgabe zugrunde, ein Verfahren zur Herstellung von Stoffgemischen bereit zu stellen, die geeignet sind zur Herstellung von Polymerwerkstoffen beispielsweise Phenolharzen, Epoxidharzen oder Formaldehydharzen und das die genannten Nachteile des Standes der Technik nicht aufweist.

Die Herstellung der Polymerwerkstoffe kann erfolgen durch die Verwendung von Stoffgemischen enthaltend Bisphenol A und Nebenprodukte, die bei der Herstellung von Bisphenol A anfallen, zur Herstellung von Polymerwerkstoffen, wie z.B. Phenolharzen, Epoxidharzen oder Formaldehydharzen.

Gegenstand der vorliegenden Erfindung ist daher ein Verfahren zur Herstellung eines Stoffgemischs enthaltend Bisphenol A und Nebenprodukte, die bei der Herstellung von Bisphenol A anfallen.

Das Stoffgemisch enthält (Angaben in Gew.-%):
p,p-BPA: 35 bis 75, bevorzugt 40 bis 65
o,p-BPA: 5 bis 25, bevorzugt 10 bis 20
Summe Bisphenole (*p,p-BPA* + *o,o-BPA*)*:* 50 bis 80, bevorzugt 60 bis 70
Nebenprodukte, die bei der Herstellung von Bisphenol A anfallen: 50 bis 20, bevorzugt 40 bis 30.

Die Summe der Gewichtsanteile aus p,p-BPA und o,p-BPA und den Nebenprodukten beträgt 100 Gew.-%.

Nebenprodukte, die bei der Herstellung von Bisphenol A anfallen, sind erfindungsgemäß Isomere des para-para-Bisphenol A, Chromane, Indane, Phenole, höhere Kondensate aus den genannten Stoffen, sowie weitere Verbindungen, deren Struktur im Einzelnen nicht geklärt ist.

Die Stoffgemische können zusätzlich zwischen 0 und 90 Gew.-%, bevorzugt 0 bis 60 Gew.-%, ganz besonders bevorzugt 0 bis 50 Gew.-% Phenol, bezogen auf die Gesamtmasse der damit entstehenden Mischung enthalten.

Eine von dieser Zusammensetzung abweichende Zusammensetzung ist für die Herstellung von z.B. Phenolharzen nachteilig. Wird beispielsweise der Anteil der hochreaktiven Komponente Phenol auf über 60 Gew.-% gesteigert, so wird in den gängigen Verfahren zur Herstellung von z.B. Platten auf Basis thermohärtender Harze, die Reaktionsgeschwindigkeit zu hoch. Das nach dem erfindungsgemäßen Verfahren hergestellte Gemisch zeigt gegenüber reinem Phenol Vorteile in der Kontrolle der Reaktionsgemschwindigkeit und dem Polymeraufbau durch gezielte Einführung von Vernetzungs- und Verzweigungssstellen.

Gegenstand der vorliegenden Erfindung sind insbesondere drei verschiedene Verfahren zur Herstellung der oben genannten Stoffgemische die in den beiden folgenden Absätzen beschrieben sind:
Verfahren zur Herstellung des oben genannten Stoffgemisches, wobei im Verfahren zur Herstellung von Bisphenol A der bei der Kristallisation und Filtration anfallenden Mutterlauge nach der Entwässerung ein Teilstrom entnommen wird, der, bevorzugt nach Abtrennung noch vorhandenen Phenols, inertisiert wird und danach abgefüllt wird.
Verfahren zur Herstellung des oben genannten Stoffgemisches, wobei im Verfahren zur Herstellung von Bisphenol A der bei der Kristallisation und Filtration anfallenden Mutterlauge nach der Entwässerung ein Teilstrom entnommen wird und einer Umlagerungsreaktion bei Temperaturen zwischen 50°C und 90°C und Verweilzeiten von 2 bis 12 h an einem sauren Ionenaustauscher zugeführt wird (ein Teil der Nebenprodukte wird dabei zu p,p-Bisphenol A umgelagert) und diesem umgelagerten Produkt ein Teilstrom entnommen wird, der, bevorzugt nach Abtrennung noch vorhandenen Phenols, intertisiert wird und danach abgefüllt wird.
Verfahren zur Herstellung des oben genannten Stoffgemisches, wobei im Verfahren zur Herstellung von Bisphenol A der bei der Kristallisation und Filtration anfallenden Mutterlauge nach der Entwässerung ein Teilstrom entnommen wird, dieser anschließend bevorzugt einer Umlagerungsreaktion bei Temperaturen zwischen 50 und 90°C und Verweilzeiten von 2 bis 12 h an einem sauren Ionenaustauscher zugeführt wird (ein Teil der Nebenprodukte wird dabei zu p,p'-Bisphenol A umgelagert) und anschließend durch destillative Verfahren aufkonzentriert wird, diesem anschließend durch Kristallisation bei einer Temperatur von 40 bis 50°C und einer Verweilzeit von 1 bis 6 Stunden ein kristallisiertes Bisphenol A Phenoladdukt entzogen und durch Filtration abgetrennt wird und das verbleibende flüssige Gemisch, bevorzugt nach Abtrennung noch vorhandenen Phenols, inertisiert und danach abgefüllt wird.

Die vorliegende Erfindung hat zahlreiche Vorteile, insbesondere haben die erfindungsgemäßen Stoffgemische eine hohe Qualität und eine gute Lagerstabilität. Sie dienen als Rohstoffe zur Herstellung hochwertiger Polymerwerkstoffe wie beispielsweise Phenolharzen, Epoxidharzen oder Formaldehydharzen.

Die Stoffgemische werden bevorzugt mit Phenol verdünnt, dabei ist das eingesetzte Phenol vorzugsweise sauerstofffrei, säurefrei, alkalifrei und metallfrei.

Die Stoffgemische werden bevorzugt unter inerten Bedingungen, d.h. insbesondere ohne Sauerstoffzutritt hergestellt.

Die Stoffgemische haben als Rohstoff für die Herstellung der genannten Polymerwerkstoffe Vorteile gegenüber reinem Bisphenol A. Beispielsweise ist die Kontrolle der Polymerisationsgeschwindigkeit einfacher, außerdem werden gezielt Vernetzungs- und Verzweigungsstellen durch die erfindungsgemäßen Stoffgemische in die genannten Polymerwerkstoffe eingeführt.

Eine Zusammensetzung der Stoffgemische die von der oben genannten Zusammensetzung abweicht, ist für die Herstellung von Polymerwerkstoffen wie beispielsweise Phenolharzen nachteilig. Wird beispielsweise der Anteil der hochreaktiven Komponente Phenol auf über 60 Gew.-%, insbesondere über 90 Gew.-% gesteigert, so wird in den gängigen Verfahren zur Herstellung von Erzeugnissen, beispielsweise Platten auf Basis thermohärtender Harze, die Reaktionsgeschwindigkeit zu hoch. Die erfindungsgemäßen Stoffgemische zeigen gegenüber reinem Phenol Vorteile in der Kontrolle der Reaktionsgeschwindigkeit und dem Polymeraufbau durch gezielte Einführung von Vernetzungs- und Verzweigungsstellen in die Polymerwerkstoffe.

Das erfindungsgemäße Verfahren zur Herstellung von BPA beruht bevorzugt auf der säurekatalysierten Umsetzung von Phenol mit Aceton, wobei bevorzugt ein Mengenverhältnis Phenol:Aceton von größer als 5 : 1 in der Reaktion eingestellt wird. Als saure Katalysatoren können homogene wie auch heterogene Brönstedsäuren oder Lewissäuren genutzt werden, so beispielsweise starke Mineralsäuren wie Salzsäure oder Schwefelsäure. Bevorzugt kommen gelförmige oder makroporöse sulfonierte vemetzte Polystyrolharze (saure Ionentauscher) zum Einsatz. Die nachfolgenden Ausführungen beziehen sich auf ein Verfahren zur Herstellung unter Nutzung von sauren Ionentauschern als Katalysatoren.

Zur Erzielung hoher Selektivitäten kann die Umsetzung von Phenol mit Aceton in Gegenwart geeigneter Mercaptoverbindungen als Cokatalysatoren durchgeführt werden. Diese können entweder homogen in der Reaktionslösung gelöst sein oder über ionische oder kovalente Bindungen an der sulfonierten Poylstyrolmatrix fixiert werden. Die Reaktionseinheit ist bevorzugt ein Schichtbett oder Wirbelbett, die auf- oder abwärts durchflossen werden, oder eine Kolonne nach Art einer Reaktivdestillationskolonne.

Bei der Umsetzung von Phenol mit Aceton in Gegenwart saurer Katalysatoren und Mercaptoverbindungen als Cokatalysatoren entsteht eine Produktmischung, die neben nicht umgesetztem Phenol und gegebenenfalls Aceton in erster Linie BPA und Wasser enthält. Daneben treten in geringen Mengen typische Nebenprodukte der Kondensationreaktion auf, so beispielsweise 2-(4-hydroxyphenyl)-2-(2-hydroxyphenyl)propan (o,p-BPA), substituierte Indene, Hydroxyphenyl-indanole, Hydroxyphenyl-chromane, substituierte Xanthene und höher kondensierte Verbindungen mit drei oder mehr Phenylringen im Molekülgerüst.

Die genannten Nebenprodukte wie auch Wasser, Phenol und Aceton können die Eignung von BPA zur Herstellung von Polymeren beeinträchtigen und müssen durch geeignete Verfahren abgetrennt werden. Insbesondere zur Herstellung von Polycarbonat werden üblicherweise hohe Reinheitsanforderungen an den Rohstoff BPA gestellt.

Die Aufarbeitung und Reingung von BPA erfolgt üblicherweise durch eine mehrstufige Kaskade von geeigneten Reinigungsverfahren wie beispielsweise Suspensionskristallisation, Schmelzekristallisation, Destillation und Desorption. In einer technisch bevorzugten Ausführungsform erfolgt die Abtrennung von BPA aus der Reaktionsmischung in Form eines etwa äquimolaren kristallinen Addukts mit Phenol durch Abkühlen der Reaktionsmischung unter Auskristallisieren des BPA/Phenol-Addukts. Die Kristallisation erfolgt bevorzugt als Suspensionskristallisation. Unter Suspensionskristallisation versteht man die Kristallisation aus einer Flüssigkeit durch Abkühlung, wobei die Kristalle mit der Flüssigkeit eine Suspension (fest-flüssig) bilden. Die BPA/Phenol-Adduktkristalle werden anschließend durch eine geeignete Apparatur zur Fest-Flüssigtrennung wie Drehfilter oder Zentrifugen von der Flüssigphase abgetrennt und erforderlichenfalls der weiteren Reinigung zugeführt. So erhaltene Adduktkristalle weisen typischerweise eine Reinheit von größer als 99 Gew.-% BPA bezogen auf die Nebenkomponenten bei einem Phenolanteil von ca. 40 Gew.-% auf. Durch Waschen mit geeigneten Lösungen, die typischerweise eine oder mehrere Komponenten aus der Gruppe Aceton, Wasser, Phenol, BPA und Nebenkomponenten enthalten, können die Adduktkristalle von oberflächlich anhaftenden Verunreinigungen befreit werden.

Der bei der Fest-Flüssigtrennung anfallende Flüssigstrom (Mutterlauge) enthält Phenol, BPA, bei der Reaktion entstandenes Wasser, nicht umgesetztes Aceton und ist angereichert an den bei der BPA-Herstellung typischerweise anfallenden Nebenkomponenten. Dieser Mutterlaugenstrom wird in einer bevorzugten Ausführungsform in die Reaktionseinheit zurückgeführt. Um die katalytische Aktivität der sauren Ionentauscher aufrecht zu erhalten wird zuvor entstandenes Wasser bevorzugt durch Destillation entfernt, wobei auch gegebenenfalls noch vorhandenes Aceton aus der Mutterlauge entfernt wird. Der so erhaltene entwässerte Reaktionsstrom wird um Phenol und Aceton ergänzt und in die Reaktionseinheit zurückgeführt. Alternativ können auch vor Durchführung der Suspensionskristallisation des BPA-Phenol-Addukts Wasser und Aceton ganz oder teilweise destillativ entfernt werden. Bei den genannten Destillationsschritten kann auch eine Teilmenge des in der Reaktionslösung vorhandenen Phenols destillativ abgetrennt werden.

Bei einer derartigen Kreislauffahrweise tritt als Problem auf, dass Nebenprodukte der BPA-Herstellung im Kreislaufstrom angereichert werden und zur Desaktivierung des Katalysatorsystem führen können. Um eine übermäßige Anreicherung von Nebenkomponenten im Kreislaufstrom zu vermeiden, wird bevorzugt eine Teilmenge des Kreislaufstroms - gegebenenfalls nach teilweiser oder vollständiger destillativer Rückgewinnung von Phenol - aus der Prozesskette als BPA-Harz ausgeschleust.

Außerdem hat es sich als vorteilhaft erwiesen einen Teil oder die Gesamtmenge des Kreislaufstroms nach der Fest-Flüssigtrennung und vor oder nach der Abtrennung von Wasser und Restaceton über eine mit saurem Ionentauscher befüllte Umlagerungseinheit zu führen. Diese Einheit wird im allgemeinen bei höheren Temperaturen betrieben als die Reaktionseinheit. In dieser Umlagerungseinheit werden unter den vorherrschenden Bedingungen einige der im Kreislaufstrom vorhandenen Nebenkomponenten der BPA-Herstellung zu BPA isomerisiert, so dass die Gesamtausbeute an BPA erhöht werden kann.

Die im Anschluss an die oben beschriebene Suspensionskristallisation der Reaktionslösung und Fest-Flüssig-Trennung erhaltenen BPA-Phenol-Adduktkristalle werden erforderlichenfalls weitergehenden Reinigungsschritten zugeführt, wobei die Abtrennung von Phenol und gegebenenfalls die Verringerung der Konzentration an Nebenkomponenten erzielt wird.

So können die Adduktkristalle beispielsweise aus Phenol, aus organischen Lösungsmitteln, aus Wasser oder Mischungen der genannten Verbindungen gemäß einer Suspensionskristallisation umkristallisiert werden. Hierbei kann durch die Wahl geeigneter Lösungsmittel auch das in den Adduktkristallen vorhandene Phenol ganz oder teilweise abgetrennt werden. Das gegebenenfalls nach der Umkristallisation im BPA verbleibende Phenol kann anschließend durch geeignete destillative, desorptive oder extraktive Methoden gänzlich abgetrennt werden.

Alternativ kann auch zunächst Phenol aus den Adduktkristallen entfernt werden. Bevorzugte Methoden hierbei sind Desorption der Schmelze mit heißen Inertgasen, Vakuumdestillation oder eine Kombination der genannten Methoden. Auf diesem Wege ist es möglich aus den Adduktkristallen BPA mit einem Restphenolgehalt von weniger als 100 ppm zu gewinnen. Durch geeignete Reaktionsführung und gegebenenfalls Zugabe von Stabilisatoren kann erreicht werden, dass BPA unter der thermischen Belastung der destillativen oder desorptiven Phenolentfernung nicht in nennenswertem Umfang gespalten wird.

In Abhängigkeit von den Verfahrensbedingungen der Suspensionskristallisation aus der Reaktionslösung und der Durchführung der Fest-Flüssigtrennung und Kristallwäsche ist das nach Abtrennung von Phenol aus den Adduktkristallen erhaltene BPA zur Herstellung von polymeren Werkstoffen geeignet. Insbesondere zur Herstellung hochwertiger Werkstoffe wie Polycarbonat kann es nötig sein, das nach Abtrennung von Phenol erhaltene BPA einer weiteren Reinigungsoperation zuzuführen. Die Endreinigung kann erfolgen durch Suspensionskristallisation aus Wasser oder geeigneten organischen Lösungsmitteln, Schmelzekristallisation in Form einer statischen oder dynamischen Schichtkristallisation, Extraktion mit Wasser, wässrigen neutralen, sauren oder basischen Salzlösungen oder geeigneten organischen Lösungmitteln oder in Form einer ein- oder mehrstufigen Destillation. Durch die Durchführung der genannten Reinigungsoperationen oder einer geeigneten Kombination derselben ist es möglich, BPA mit einer Reinheit von größer als 99,9 Gew.-% zu erhalten, das zur Herstellung hochwertiger Polymerwerkstoff in besonderer Weise geeignet ist.

Eine bevorzugte Ausführungsform der vorliegenden Erfindung ist dadurch gekennzeichnet, dass der im beschriebenen Verfahren zur Herstellung von Bisphenol A bei der Kristallisation und Filtration anfallenden Mutterlauge nach der Entwässerung ein Teilstrom entnommen wird. Dieser Teilstrom entspricht bevorzugt 5 bis 15 Gew.-% der anfallenden Mutterlauge. Dieser Teilstrom enthält vorzugsweise weniger als 0,5 Gew.-% Wasser und vorzugsweise weniger als 0,1 Gew.-% Aceton. Zur Herstellung des erfindungsgemäßen Stoffgemisches wird der genannte Teilstrom bevorzugt filtriert, von Säurespuren bevorzugt vollständig befreit, danach in jedem Fall inertisiert und bei Temperaturen von bevorzugt 60 bis 100°C in Gefäßen, die bevorzugt aus Edelstahl bestehen, abgefüllt.

Eine weitere bevorzugte Ausführungsform der vorliegenden Erfindung ist dadurch gekennzeichnet, dass ein nach dem im vorigen Absatz beschriebenen Verfahren erzeugter Teilstrom bei Temperaturen von bevorzugt 50 bis 70°C, insbesondere 60 bis 70°C, besonders bevorzugt 65°C, an einem sauren Ionenaustauscher isomerisiert wird. Dabei werden isomerisierbare Bestandteile beispielsweise o, p-Bisphenol A, zu p,p-Bisphenol A isomerisiert. Die Verweilzeit im Isomerisierungsreaktor beträgt bevorzugt 2 bis 12 Stunden, besonders 3 bis 8 Stunden, besonders bevorzugt 3 bis 4 Stunden. Zur Herstellung des erfindungsgemäßen Stoffgemisches wird der Teilstrom nach der Isomerisierung filtriert, von Säurespuren bevorzugt vollständig befreit, in jedem Fall inertisiert und bei Temperaturen von bevorzugt 60 bis 100°C in Gefäße, bevorzugt aus Edelstahl, abgefüllt.

Eine weitere bevorzugte Ausführungsform der vorliegenden Erfindung ist dadurch gekennzeichnet, dass der im beschriebenen Verfahren zur Herstellung von Bisphenol A bei der Kristallisation und Filtration anfallenden Mutterlauge nach der Entwässerung ein Teilstrom entnommen wird. Dieser Teilstrom entspricht vorzugsweise 5 bis 15 % der anfallenden Mutterlauge. Dieser Teilstrom enthält vorzugsweise unter 0,5 Gew.-% Wasser und unter 0,1 Gew.-% Aceton. Der Teilstrom wird dann einer Umlagerungsreaktion bei Temperaturen zwischen 50 und 90°C und Verweilzeiten von 2 bis 12 h an einem sauren Ionenaustauscher umgesetzt. Der Teilstrom wird dann durch destillative Verfahren aufkonzentriert. Hierbei wird im wesentlichen Phenol abgetrennt. Der Phenolgehalt nach der Aufkonzentrierung beträgt bevorzugt weniger als 70 Gew.-%, insbesondere weniger als 60 Gew.-%. Das so aufkonzentrierte Gemisch wird in einer bevorzugt 1- bis 2-stufigen Kristallisation bei einer Temperatur von 40 bis 50°C und einer Verweilzeit von 1 bis 6 Stunden kristallisiert. Das so erhaltene kristallisierte Bisphenol A-Phenol-Addukt wird durch Filtration abgetrennt und dem Hauptprozess der Bisphenol A-Herstellung zugeführt. Das verbleibende flüssige Gemisch oder ein Teil hiervon wird gegebenenfalls nach weiterer Aufkonzentrierung, wobei weiteres Phenol abgetrennt wird, zur Herstellung des erfindungsgemäßen Stoffgemisches bevorzugt filtriert, von Säurespuren vorzugsweise vollständig befreit, in jedem Fall inertisiert und bei Temperaturen von bevorzugt 80 bis 125°C in Gefäße, die bevorzugt aus Edelstahl bestehen, abgefüllt.

Die nach dem erfindungsgemäßen Verfahren hergestellten Stoffgemische enthalten bevorzugt zusätzlich Phenol in einem Anteil von 0 bis 90 Gew.-%, insbesondere 0 bis 60 Gew.-%, besonders bevorzugt 0 bis 50 Gew.-%

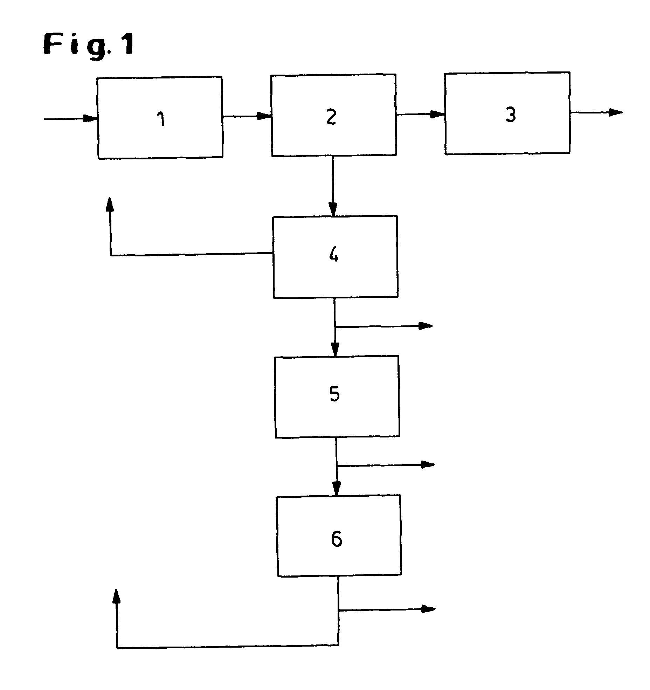

Die Zeichnung Fig. 1 stellt schematisch ein Verfahren zur Herstellung der erfindungsgemäßen Stoffgemische dar. Die Zeichnung repräsentiert eine bevorzugte Ausführungsförm der Erfindung; die Erfindung ist in ihrem Umfang nicht auf die Zeichnung beschränkt.

Der Einheit 1 werden Phenol und Aceton zugeführt, die in Einheit 1 zu Bisphenol A reagieren. In Einheit 2 findet die Kristallisation des Adduktes aus Bisphenol A und Phenol statt. Außerdem findet in Einheit 2 die Abtrennung des kristallisierten Adduktes durch Filtration statt. Das abgetrennte Addukt wird Einheit 3 zugeführt in der die Abtrennung und Aufarbeitung des Bisphenol A erfolgt. Die verbleibende Mutterlauge wird aus Einheit 2 in Einheit 4 überführt, in der die Abtrennung von Wasser (Entwässerung) erfolgt. Aus Einheit 4 wird ein Anteil von 85 bis 95 Gew.-% unter Zuführung von 2 bis 6 Gew.-% Aceton in Einheit 1 zurückgeführt. Nach Einheit 4 kann ein erfindungsgemäßes Stoffgemisch abgetrennt werden. Der kleinere Teil (5 bis 15 Gew.-%) der in Einheit 4 entwässerten Mutterlauge wird Einheit 5 zugeführt. In Einheit 5 findet die Umlagerung statt. Nach Einheit 5 kann ein erfindungsgemäßes Stoffgemisch abgetrennt werden. Die in Einheit 5 umgelagerten Mutterlauge wird Einheit 6 zugeführt. In Einheit 6 findet die Abtrennung von Phenol die Kristallisation und die Abtrennung von Bisphenol A/Phenol Addukten als Feststoffe durch Filtration statt. Nach Einheit 6 kann aus der verbleibenden Mutterlauge gegebenenfalls nach Abtrennung noch vorhandenen Phenols durch bekannte Verfahren wie z.B. Destillation, Desorption etc. ein erfindungsgemäßes Stoffgemisch abgetrennt werden. Das wiedergewonnene Phenol wird aus Einheit 6 unter Zuführung von 2 bis 6 Gew.-% Aceton der Einheit 1 zugeführt. Die erfindungsgemäßen Stoffgemische, die nach Einheit 4, 5 oder 6 abgetrennt werden, werden der Abfüllung zugeführt.

Im Folgenden wird die Erfindung durch Beispiele veranschaulicht, ohne in ihrem Umfang auf die Beispiele beschränkt zu sein.

Im Folgenden werden erfindungsgemäße Stoffgemische und deren Herstellung beispielhaft dargelegt. Die Herstellung erfolgte jeweils in einer Anordnung wie sie in Fig. 1 beschrieben ist.

Die angegebenen Zusammensetzungen der erfindungsgemäßen Stoffgemische beziehen sich auf mehrere Chargen die von jedem Stoffgemisch hergestellt wurden. Bei jeder einzelnen Charge betrug die Summe alle Komponente 100 Gew.-%.

Nach Einheit 4 wurde ein erfindungsgemäßes Stoffgemisch (BPG 1) abgetrennt.

Nach Einheit 5 wurde ein erfindungsgemäßes Stoffgemisch (BPG 2) abgetrennt.

Nach Einheit 6 wurde ein erfindungsgemäßes Stoffgemisch (BPG 3) abgetrennt.

| BPG1 | |
|---|---|
| p,p-BPA | 50 bis 60 Gew.-% |
| o,p-BPA | 10 bis 20 Gew.-% |
| Summe Bisphenole¹⁾ | 65 bis 75 Gew.-% |
| Trisphenole | 0 bis 5 Gew.-% |
| Indane | 0 bis 10 Gew-% |
| Chromane | 5 bis 15 Gew.-% |
| Restkomponenten²⁾ | 30 bis 2 Gew.-% |

| BPG2 | |
|---|---|
| p,p-BPA | 60 bis 70 Gew.-% |
| o,p-BPA | 5 bis 15 Gew.-% |
| Summe Bisphenole¹⁾ | 65 bis 75 Gew.-% |
| Trisphenole | 0 bis 3 Gew.-% |
| Indane | 0 bis 10 Gew-% |
| Chromane | 5 bis 15 Gew.-% |
| Restkomponenten²⁾ | 30 bis 2 Gew.-% |

| BPG3 | |
|---|---|
| p,p-BPA | 35 bis 45 Gew.-% |
| o,p-BPA | 10 bis 20 Gew.-% |
| Summe Bisphenole¹⁾ | 55 bis 65 Gew.-% |
| Trisphenole | 0 bis 5 Gew.-% |
| Indane | 5 bis 15 Gew-% |
| Chromane | 15 bis 25 Gew.-% |
| Restkomponenten²⁾ | 30 bis 2 Gew.-% |

| | |
|---|---|
| 1*) p, p-BPA + o, o-BPA* | |
| *2) Alle Komponenten außer Phenol und Bisphenole (p,p-BPA + o,p-BPA + o,o- BPA). Die Restkomponenten bestehen beispielsweise aus verschiedenen Phe- nolen, höhermolekularen Kondensaten, Isopropenylphenolen etc.)* | |

Im Folgenden werden Vergleichsbeispiele aufgeführt:
BPG4 wurde erhalten wie BPG1, jedoch ohne Entfernung von Säure bzw. Säurespuren. Der Wassergehalt von BPG4 war größer als 0,5 Gew.-%. Nach einer Standzeit von mehr als 5 Tagen zersetzte sich BPG4, das anfänglich einen Gehalt an p,p-BPA von 50,2 Gew.-% und einen Gehalt an Restkomponenten von 29,4 Gew.-% aufwies. Nach der Zersetzung war die Zusammensetzung von BPG4 47,7 Gew.-% p,p-BPA und 32,2 Gew.-% Restkomponenten.
BPG5 wurde erhalten wie BPG2, das Edukt für die Umlagerung war dabei ein Gemisch aus 50,2 Gew.-% p,p-BPA und 8,2 Gew.-% Indanen. Die Umlagerung erfolgte unter ungeeigneten Bedingungen bei 80°C und 15 Stunden. Das Resultat war eine erhöhte Indanbildung unter Zersetzung von p,p-BPA. Nach der Umlagerung unter ungeeigneten Bedingungen hatte BPG5 einen Gehalt an p,p-BPA von 42,7 Gew.-% und einen Indangehalt von 15,0 Gew.-%.
BPG6 wurde erhalten wie BPG3, jedoch wurden keine inertisierten Bedingungen angewandt. Nach einer Standzeit von über 5 Tagen bei Umgebungsbedingungen wurde eine deutliche Farbverschlechterung ausgedrückt als lodfarbzahl erhalten. Die Iodfarbzahl erhöhte sich von 300 auf über 1 000. Zusätzlich verringerte sich der Gehalt an p,p-BPA durch Zersetzung.

## Patentansprüche

1. Verfahren zur Herstellung eines Stoffgemisches enthaltend
35 bis 75 Gew.-% p,p-Bisphenol A und
5 bis 25 Gew.-% o,p-Bisphenol A und
20 bis 50 Gew.-% Nebenprodukte, die bei der Herstellung von Bisphenol A durch säurekatalysierte Umsetzung von Phenol mit Aceton anfallen,
wobei die Summe der Gewichtsanteile aus p,p-Bisphenol A und o,p-Bisphenol A 50 bis 80 Gew.-% beträgt, und wobei die Summe der Gewichtsanteile aus p,p-Bisphenol A und o,p-Bisphenol A und den Nebenprodukten 100 Gew.-% beträgt,
wobei in einem Verfahren zur Herstellung von Bisphenol A durch säurekatalysierte Umsetzung von Phenol mit Aceton, das eine Kristallisation eines Bisphenol A - Phenol - Addukts umfasst, und das eine Filtration zur Abtrennung der Bisphenol A - Phenol - Adduktkristalle von einer Mutterlauge umfasst, dieser bei der Kristallisation und Filtration anfallenden Mutterlauge nach einer Entwässerung ein Teilstrom entnommen wird,
und wobei dieser Teilstrom von Säurespuren befreit wird, inertisiert wird und danach abgefüllt wird.

2. Verfahren nach Anspruch 1, bei dem aus dem Teilstrom zunächst noch vorhandenes Phenol abgetrennt wird, und der Teilstrom anschließend inertisiert und danach abgefüllt wird.

3. Verfahren nach Anspruch 1, bei dem der Teilstrom zunächst an einem sauren Ionenaustauscher bei 50-90°C bei Verweilzeiten von 2 bis 12 Stunden isomerisiert wird, und anschließend durch destillative Verfahren aufkonzentriert wird, und aus diesem Teilstrom anschließend bei einer Temperatur von 40 bis 50°C und einer Verweilzeit von 1 bis 6 Stunden ein Bisphenol-A-Phenol-Addukt auskristallisiert und aus dem Teilstrom durch Filtration abgetrennt wird, und wobei das danach verbleibende flüssige Gemisch inertisiert und danach abgefüllt wird.

4. Verfahren nach Anspruch 3, bei dem aus dem danach verbleibenden Gemisch zunächst noch vorhandenes Phenol abgetrennt wird, und das danach verbleibende flüssige Gemisch anschließend inertisiert und danach abgefüllt wird.

5. Verfahren nach einem der Ansprüche 1 bis 4, bei dem die Stoffgemische mit Phenol verdünnt werden.

6. Verfahren nach einem der Ansprüche 1 - 5, bei dem die Stoffgemische Phenol in einem Anteil von 0 bis 90 Gew.-% enthalten.

7. Verfahren nach einem der Ansprüche 3 bis 6, bei dem die Isomerisierung bei höheren Temperaturen durchgeführt wird als die säurekatalysierte Umsetzung von Phenol mit Aceton.

## Claims

1. A process for preparing a mixture of substances containing
35 to 75 wt.% of p,p-bisphenol A and
5 to 25 wt.% of o,p-bisphenol A and
20 to 50 wt.% of secondary products which are produced during the preparation of bisphenol A by acid-catalysed reaction of phenol with acetone,
wherein the sum of the proportions by weight of p,p-bisphenol A and o,p-bisphenol A is 50 to 80 wt.% and wherein the sum of the proportions by weight of p,p-bisphenol A and o,p-bisphenol A and the secondary products is 100 wt.%,
wherein, in a process for preparing bisphenol A by acid-catalysed reaction of phenol with acetone which includes the crystallisation of a bisphenol A - phenol adduct and which includes a filtration process to separate the bisphenol A - phenol adduct crystals from a mother liquor, a substream is taken from this mother liquor produced during crystallisation and filtration, after dewatering,
and wherein traces of acid are removed from this substream, the substream is rendered inert and is then put into containers.

2. A process according to Claim 1 in which any phenol still present is first separated from the substream and the substream is subsequently rendered inert and then put into containers.

3. A process according to Claim 1, in which the substream is first isomerised on an acid ion-exchanger at 50-90°C with residence times of 2 to 12 hours and is then concentrated by distillation and a bisphenol A - phenol adduct is then crystallised out of this substream at a temperature of 40 to 50°C with a residence time of 1 to 6 hours and is separated from the substream by filtration, and wherein the liquid mixture remaining after that is rendered inert and then put into containers.

4. A process according to Claim 3, in which any phenol still present is first separated from the mixture remaining after that and the liquid mixture remaining after that is subsequently rendered inert and is then put into containers.

5. A process according to one of Claims 1 to 4, in which the mixtures of substances are diluted with phenol.

6. A process according to one of Claims 1 - 5, in which the mixtures of substances contain phenol in a proportion of 0 to 90 wt.%.

7. A process according to one of Claims 3 to 6, in which isomerisation is performed at higher temperatures than the acid-catalysed reaction of phenol with acetone.

## Revendications

1. Procédé de fabrication d'un mélange de substances contenant
35 à 75 % en poids de p,p-bisphénol A et
5 à 25 % en poids de o,p-bisphénol A et
20 à 50 % en poids de sous-produits qui sont formés lors de la fabrication de bisphénol A par réaction catalysée en milieu acide du phénol avec l'acétone,
la somme des parties en poids de p,p-bisphénol A et o,p-bisphénol A étant de 50 à 80 % en poids, et la somme des parties en poids de p,p-bisphénol A et o,p bisphénol A et des sous-produits étant de 100 % en poids,
dans un procédé de fabrication de bisphénol A par réaction catalysée en milieu acide du phénol avec l'acétone, qui comprend une cristallisation d'un produit d'addition de bisphénol A et de phénol et qui comprend une filtration pour la séparation d'avec une eau mère des cristaux de produit d'addition de bisphénol A et de phénol, un flux partiel étant prélevé de l'eau mère formée au cours de la cristallisation et de la filtration après la déshydratation,
et ce flux partiel étant débarrassé des traces d'acide, inertisé et ensuite soutiré.

2. Procédé suivant la revendication 1, pour lequel on sépare d'abord du flux partiel le phénol encore présent et le flux partiel est ensuite inertisé et après cela soutiré.

3. Procédé suivant la revendication 1, pour lequel le flux partiel est d'abord isomérisé dans un échangeur d'ions acide à 50-90°C avec des temps de séjour de 2 à 12 heures, et ensuite concentré par un procédé de distillation, et un produit d'addition de bisphénol A et de phénol est ensuite cristallisé à partir de ce flux partiel à une température de 40 à 50°C et avec un temps de séjour de 1 à 6 heures et séparé du flux partiel par filtration, et le mélange liquide restant ensuite étant inertisé et puis soutiré.

4. Procédé suivant la revendication 3, pour lequel on sépare d'abord du mélange restant le phénol encore présent et le mélange liquide restant est ensuite inertisé et puis soutiré.

5. Procédé suivant l'une des revendications 1 à 4, pour lequel les mélanges de substances sont dilués avec du phénol.

6. Procédé suivant l'une des revendications 1 à 5, pour lequel les mélanges de substances ont une teneur en phénol de 0 à 90 % en poids.

7. Procédé suivant l'une des revendications 3 à 6, pour lequel l'isomérisation est réalisée à des températures plus élevées que la réaction catalysée en milieu acide du phénol avec l'acétone.
